# EUROPÄISCHE PATENTSCHRIFT

(11) **EP 1 634 945 B1**
(45) Veröffentlichungstag und Bekanntmachung des Hinweises auf die Patenterteilung: **27.02.2008**
(21) Anmeldenummer: 05018079.3
(22) Anmeldetag: 19.08.2005
(51) Int. Cl.: C12M 1/00, B01L 7/00, C12M 1/12

(54) **Lufterwärmungsvorrichtung für Proben**
Air-conditioned cabinet for samples
Armoire de climatisation pour échantillon

(30) Priorität: 10.09.2004 DE 102004043909
(43) Veröffentlichungstag der Anmeldung: 15.03.2006
(73) Patentinhaber: Thermo Electron LED GmbH, 63505 Langenselbold (DE)
(72) Erfinder: Heeg, Hubert, 63776 Mömbris (DE); Ferger, Stefan, 63691 Ranstadt (DE); Ross, Gerd, 60388 Frankfurt (DE)
(74) Vertreter: Lang, Friedrich

(56) Entgegenhaltungen:
- EP-A- 1 442 794
- GB-A- 926 541
- US-A- 4 250 266
- US-A- 4 868 122
- US-A- 5 360 741
- US-A1- 2002 047 311

## Beschreibung

Die Erfindung betrifft eine Erwärmungsvorrichtung für Proben auf dem Gebiet der Life-Science mit mindestens einer Kassette mit horizontal ausgerichteten Lagerfächern zur Lagerung von Probenträgern, bei welcher Gas zur Erwärmung der Proben auf die Kassette geführt ist, mit einem Gehäuse mit einem darin angeordneten Lagerraum, der zur Zu- und Abfuhr von Gas und zur Lagerung der Kassetten ausgebildet ist, und mit einer Gasumwälzungseinrichtung zur Umwälzung des Gases im Innenraum sowie ein Verfahren zur Erwärmung solcher Proben. Unter dem Begriff Life-Science versteht man allgemein "die Wissenschaft vom Leben", welche sich mit der Gewinnung und Anwendung von wissenschaftlichen Kenntnissen zum Beispiel auf den Gebieten der Chemie, der Biologie, der Pharmazie, der Biotechnologie, der Physik und der Biochemie beschäftigt. Im Rahmen der wissenschaftlichen Forschung auf diesem Gebiet ist es häufig notwendig, Proben, die üblicherweise in Probenträgern, insbesondere in Mikrotiterplatten, gelagert werden, zu erwärmen. Häufig ist es wünschenswert, die Proben auf eine bestimmte Temperatur zu erwärmen. Ein typisches Anwendungsbeispiel ist das Auftauen von gefrorenen Proben.

Üblicherweise werden die Probenträger in Kassetten gelagert, welche horizontal ausgerichtete Lagerfächer zur Aufnahme der Probenträger aufweisen. Die Lagerfächer sind normalerweise übereinander angeordnet und jedes Fach weist mindestens eine Öffnung zur Einführung der Probenträger auf. Ferner weist jedes Fach mindestens eine weitere Öffnung auf, so dass das die Kassetten umgebene Gas durch die Fächer hindurch strömen kann.

Es ist bekannt, dass Proben zur Auftauung der Umgebungstemperatur ausgesetzt werden oder erwärmte Luft aus der Umgebung auf die mit Proben bestückten Kassetten geleitet wird. Dabei kann allerdings eine für alle Proben gleichmäßiger Temperaturverlauf nicht immer sichergestellt werden. Aus der US 4,868,122 A ist auch bekannt, dass Gas im Lagerraum von Vorrichtungen zur Züchtung von Dauerformen, wie z.B. filamentöser Mikroorganismen, mit einer Gasumwälzungseinrichtung umzuwälzen. Die Vorrichtung der US 4,868,122 A ist allerdings sehr aufwendig im Betrieb. Durch den hohen Bedienungsaufwand ist diese Vorrichtung zur Erwärmung von Proben auf dem Gebiet der Life-Scienic ungeeignet. Ferner erlaubt die Vorrichtung nur einen chargeweisen Betrieb bzw. keinen kontinuierlichen Probenaustausch, so dass zeit- und kostenintensive Nutzungspausen auftreten.

Es ist Aufgabe der vorliegenden Erfindung, eine Erwärmungsvorrichtung sowie ein Verfahren zur Erwärmung von Proben anzugeben, bei der eine kontrollierte und gleichmäßige Erwärmung der Proben erreicht wird.

Die Lösung dieser Aufgabe gelingt mit einer Erwärmungsvorrichtung und mit einem Verfahren gemäß einem der unabhängigen Ansprüche. Vorteilhafte Weiterbildungen sind in den Unteransprüchen angegeben.

Durch das Vorhandensein eines Gehäuses, durch den der Lagerraum von der Umwelt abschließbar ist, werden die im Lagerraum gelagerten Proben nicht der Umgebung ausgesetzt und die Gefahr einer Kontamination kann somit reduziert werden. Die Proben können dabei manuell oder durch ein automatisiertes Be- und Entladungssystem in den Lagerraum eingeführt werden. Ein solche automatisierte Be- und Entladungsvorrichtung ist zum Beispiel aus der US 6,129,428 bekannt. Nach Einlagerung der Proben in den Lagerraum wird das Gehäuse verschlossen und der Lagerraum somit gegenüber der Umwelt abgeschottet.

Ein weiterer Vorteil der Erfindung liegt darin, dass es durch die Ausbildung des Lagerraums zur Zu- und Abfuhr von Gas möglich ist, Gas zur Erwärmung der Proben auf die Kassetten zu führen. Durch die geschlossenen Anordnung des Lagerraumes, ist es möglich, die Gasströmung zu steuern und somit eine gleichmäßigere Erwärmung der Proben zu erreichen. Durch die Gasumwälzeinrichtung ist es weiterhin möglich, nur das bereits im Lagerraum vorhandene Gas zur Erwärmung der Proben zu benutzen, ohne somit eine Kontamination aus der Umgebungsluft zu riskieren. Mit der Erfindung ist es möglich, das Erwärmen , insbesondere Auftauen, der Proben mit kontrollierter Geschwindigkeit, gleichmäßig und schonend durchzuführen, so dass eine optimale Weiterverarbeitung der Proben möglich ist, ohne dass die Proben durch den Erwärmungsvorgang kontaminiert oder beschädigt werden.

Die Öffnung des Gehäuses ist so anzuordnen, dass sie einen ausreichenden Zugriff auf den Lagerraum gewährt und somit das einfache Ein- und Ausladen von Kassetten in den Lagerraum ermöglicht. Der Lagerraum kann so ausgebildet sein, dass er entweder den gesamten Bereich, der vom Gehäuse umschlossen ist, ausfüllt oder nur einen Teil dieses Bereichs. Es werden mehrere Kassetten innerhalb des Lagerraums gelagert. Weiterhin sind die Kassetten mittels Bewegungsmitteln ortsverschiebbar ausgebildet und mittels einer Steuereinrichtung in Richtung der Gasführung bewegbar. Durch die Bewegung der Kassetten in Richtung der Gasführung ist es möglich, die Kassetten abwechselnd so anzuordnen, dass eine Durchströmung der Kassetten erreicht wird. Durch die Steuereinrichtung sind die Bewegungen der Kassetten so einstellbar, dass eine in etwa gleichmäßige Durchströmung aller Kassetten gewährleistet ist. Die Bewegungsmittel umfassen eine horizontal verschwenkbare oder rotierbare Kassettenlagerplattform. Die Bewegungsmittel umfassen daher eine Transporteinrichtung, die zum Transport von einzelnen Probenträgern senkrecht und parallel zur Rotationsachse der Drehscheibe ausgebildet ist. Durch Öffnen der Gehäuseöffnung wird der Zugriff auf den Lagerraum gewährt und die Kassetten können manuell in den Lagerraum eingebracht werden. Die Kassetten werden auf der Drehscheibe angeordnet und können somit durch Drehung in Richtung der Gasführung bewegt werden. Die Transporteinrichtung, welche bereits aus der US 6,129,428 bekannt ist, weist ein schwenkbar und horizontal verschiebbar ausgebildetes Transportelement auf, durch welches ein Probenträger zur Zeit transportierbar ist. Das Transportelement ist in einen Lift integriert, durch welchen die ein- und auszulagernden Probenträger auch vertikal bewegbar sind. Durch eine zusätzliche, verschließbare Be- und Entladungsöffnung im Gehäuse, dessen Größe an die Größe der Probenträger angepasst ist, kann ein Probenträger zur Zeit von der Transporteinrichtung aufgenommen werden und in ein Lagerfach einer Kassette, welche auf der Drehscheibe angeordnet ist, eingebracht werden. Durch eine Schleusenvorrichtung in der Be- und Entladungsöffnung kann eine Kontamination des Innenraums des Gehäuses beim Be- und Entladen vermieden werden.

In einer bevorzugten Ausführungsform weist die Gasumwälzungseinrichtung eine Zuführung auf, durch die externes Gas zum Umwälzkreislauf zugeführt werden kann. Zusätzlich weist die Gasumwälzungseinrichtung auch eine Abführung auf, durch die Gas aus dem Umwälzkreislauf abgeführt und in die Umgebungsluft außerhalb des Gehäuses der Erwärmungsvorrichtung abgegeben werden kann. Dies hat den Vorteil, dass das Gas innerhalb des Umwälzkreislaufes durch externes Gas ersetzt und somit erneuert werden kann. Hierbei ist es möglich, das Gas im Umwälzkreislauf komplett auszutauschen oder schrittweise externes Gas zuzuführen und fortlaufend einen Teil des Gasgemisches, bestehend aus dem ursprünglichen Gas und dem neu zugeführten externen Gas, aus dem Umwälzkreislauf abzulassen. Durch Einstellung der Zu- und Abführmengen von Gas aus dem Umwälzkreislauf ist es weiterhin möglich, den Gasdruck innerhalb des Umwälzkreislaufes zu variieren und somit an die jeweiligen Proben angepasste Umweltbedingungen zu erzeugen.

In einer weiteren bevorzugten Ausführungsform weist die Gasumwälzungseinrichtung Mittel zur Aufbereitung des umzuwälzenden Gases auf. Unter umzuwälzendem Gas wird das gesamte Gas im Umwälzkreislauf, das heißt sowohl das im Lagerraum als auch das in der Gasumwälzeinrichtung befindliche Gas, verstanden. Durch die Mittel zur Aufbereitung ist es möglich, physikalische oder chemische Eigenschaften des umzuwälzenden Gases zu verändern und somit optimale Bedingungen für den Erwärmungsprozess der jeweiligen Proben zu schaffen.

Zeckmäßigerweise weist die Gasumwälzungseinrichtung eine Gasführung auf. Unter Gasführung kann dabei grundsätzlich jedes Medium verstanden werden, das zur Führung von Gas geeignet ist, beispielsweise Leitungen, Rohre, Wände, etc. Die Gasführung ist dabei so ausgebildet, dass sie das Gas entlang der gesamten Länge der mindestens einen Kassette führt und in im Wesentlichen horizontaler Ausrichtung in die Kassette zur Durchströmung einleitet. Die Führung des Gases entlang der gesamten Länge der Kassette stellt sicher, dass das Gas in allen Bereichen der Kassette weitestgehend gleichmäßig verteilt ist und so eine gleichförmige Durchströmung und daraus resultierend auch eine gleichmäßige Erwärmung der Proben in der Kassette sichergestellt wird. Die Gasführung wird zweckmäßigerweise so angeordnet, dass das Gas zumindest entlang einer Seite der Kassette geführt wird, welche Öffnungen aufweist und so eine Durchströmung der Kassette mit Gas erlaubt. Die horizontale Ausrichtung der Strömung des eingeleiteten Gases in die Kassette stellt weiterhin sicher, dass sämtliche Lagerfächer durchströmt werden, da diese ebenfalls horizontal ausgerichtet sind. Die Gasführung kann sowohl innerhalb als auch außerhalb des Lagerraums angeordnet sein. Falls sie außerhalb des Lagerraums angeordnet ist, ist die Zuführung des Gases von der Gasführung in den Lagerraum so anzuordnen und auszubilden, dass eine ausreichende Durchströmung der mindestens einen Kassette gewährleistet ist.

In einer weiteren bevorzugten Ausführungsform wird die Gasführung als Schacht, insbesondere als rechteckiger Schacht ausgebildet, wobei der Schacht an den Lagerraum angrenzt und sich über die Länge des Lagerraumes erstreckt. Da schrankartige Gehäuse, wie sie auch für die erfindungsgemäße Erwärmungsvorrichtung verwendet werden können, häufig quaderförmig ausgebildet sind, bietet es sich an, den Gasführungsschacht mit rechteckigem Querschnitt auszubilden. Bevorzugt grenzt der Schacht in der Nähe der mindestens einen Kassette an den Lagerraum an, so dass das Gas nach Einführung in den Lagerraum von der Gasführung aus, direkt auf die mindestens eine Kassette geleitet werden kann. Zweckmäßigerweise wird die Kassette hierbei mit ihren Öffnungen zur Zuführung in den Lagerraum ausgerichtet. Vorteilhaft ist, dass ein Schacht vergleichsweise einfach ausgebildet werden kann, beispielsweise kann er aus den Wänden des Gehäuses und/oder des Lagerraumes gebildet werden. Das sich der Gasführungsschacht über die gesamte Länge des Lagerraums erstreckt, gewährleistet, dass eine gleichmäßige Durchströmung des Lagerraums und somit auch der mindestens einen Kassette mit Gas erreicht wird.

Bevorzugterweise ist die Gasumwälzungseinrichtung in das Gehäuse integriert ausgebildet. Dabei kann die Gasumwälzungseinrichtung sowohl innerhalb als auch außerhalb des Lagerraums angeordnet sein. Es ist auch möglich, nur einen Teil der Gasumwälzungseinrichtung innerhalb des Lagerraums und einen anderen Teil außerhalb, also zwischen Lagerraum und Gehäuse, anzuordnen. Vorteilhaft ist hierbei, dass durch die Integrierung der Gasumwälzungseinrichtung in das Gehäuse eine kompakte Bauweise der Erwärmungsvorrichtung verwirklicht werden kann. Alternativ ist die Gasumwälzungseinrichtung separat und auf das Gehäuse aufsetzbar ausgebildet, wobei es in dieser Ausführungsform mit Befestigungsmitteln, bevorzugterweise mit Scharnieren, Schrauben, Bolzen oder durch Verschweißen, am Gehäuse angebracht wird. Dies hat den Vorteil, dass die Gasumwälzungseinrichtung separat vom Rest der Erwärmungsvorrichtung gefertigt werden kann und die Endmontage erst unmittelbar vor Inbetriebnahme unternommen werden muss. Weiterhin ist es hierdurch relativ leicht möglich, andere in Laboren weit verbreitete Vorrichtungen, wie zum Beispiel Klimaschränke, Kühlschränke, Lagerungsvorrichtungen, Lagerschränke für Proben, etc., umzurüsten und zu erfindungsgemäßen Erwärmungsvorrichtungen auszubilden.

Bei einer auf das Gehäuse aufsetzbar ausgebildeten Gasumwälzungseinrichtung, wird diese bevorzugterweise im Bereich der Öffnung des Gehäuses angeordnet und mittels Scharnieren am Gehäuse zur schwenkbaren Verschließung der Öffnung befestigt. Durch die Befestigung mittels Scharnieren kann die Gasumwälzungseinrichtung, ähnlich einer Tür, seitlich auf- bzw. zugeschwenkt werden und ermöglicht so den Zugang zum Lagerraum zur Be- bzw. Entladung von Kassetten. Zweckmäßigerweise schließt die Gasumwälzungseinrichtung die Öffnung des Gehäuses dichtend ab, so dass ein Austritt von Gas aus dem Inneren des Gehäuses vermieden wird.

Bevorzugterweise wird die Gasführung versetzt von der Öffnung des Gehäuses angeordnet. Dies ist allerdings nur bevorzugt, solange die Gasumwälzungseinrichtung nicht als Tür zur Verschließung der Öffnung des Gehäuses ausgebildet ist. Vorteilhaft ist diese Anordnung der Gasführung deshalb, da dadurch die Be- und Entladung des Lagerraums nicht beeinträchtigt wird. Zweckmäßigerweise weist die Gasumwälzungseinrichtung einen Antrieb zur Umwälzung des Gases auf. Besonders bevorzugt ist der Antrieb als Gebläse ausgebildet. Das Gebläse wird dabei typischerweise so angeordnet, dass es das Gas innerhalb des Umwälzkreislaufes auf einer Seite ansaugt und auf der anderen ausbläst und so für eine kontinuierliche Strömung sorgt.

Sind in allen Kassetten gleiche Proben vorhanden, ist es zweckmäßig die Kassetten kontinuierlich in Richtung der Gasführung zu bewegen, so dass sich für jede Kassette eine etwa gleiche Durchströmdauer einstellt. Bei unterschiedlichen Proben (zum Beispiel unterschiedliche Inhalte, unterschiedliche Temperaturen, unterschiedliche Menge, etc.) ist möglich, die Steuereinrichtung so einzustellen, dass die jeweiligen Kassetten unterschiedlich lange in der Nähe der Gasführung verweilen, abhängig davon, welche Proben wieviel Zeit zur Erwärmung benötigen. Hierdurch kann die Gleichmäßigkeit der Erwärmung der Proben weiter verbessert werden. Es ist weiterhin zweckmäßig, dass die Steuereinrichtung die Verweilzeit der Kassetten in der Nähe der Gasführung in Abhängigkeit der Strömungsgeschwindigkeit des auf die Kassetten geführten Gases steuert. Die Abhängigkeit der Verweildauer der Kassetten von der Strömungsgeschwindigkeit des auf die Kassetten geführten Gases stellt sicher, dass bei jeder Strömungsgeschwindigkeit eine ausreichende Durchströmung der Kassetten erreicht wird.

Vorteilhafterweise ist die Kassettenlagerplattform als Drehscheibe, die um ihren Mittelpunkt rotierbar ist, ausgebildet. Diese Ausbildungsform wird im Allgemeinen auch als "Karussell" bezeichnet.

Vorteilhafterweise sind die Kassetten auf der Transportplattform im Wesentlichen gleichmäßig verteilt. Dadurch wird die Gleichmäßigkeit der Erwärmung der Proben weiter verbessert.

Bevorzugterweise wird das externe Gas dem Umwälzkreislauf unter einem bestimmten Mischungsverhältnis beigemischt. Hierdurch kann eine konstante und kontinuierliche Beimischung von externem Gas zum Umwälzkreislauf erreicht werden und die Gasmischung so auf die jeweiligen Erfordernisse der Proben eingestellt werden.

In einer weiteren bevorzugten Ausführungsform weist die Zuführung des externen Gases ein Schadstoff- und/oder Keimfilter zur Filterung des externen Gases auf. Durch die Filterung des externen Gases wird sichergestellt, dass durch die Zuführung von fremden Gas in den Umwälzkreislauf keine Schadstoffe und Keime in den Lagerraum gelangen und eine Kontamination der Proben somit vermieden wird.

In einer weiteren Ausführungsform weisen die Mittel zur Aufbereitung des umzuwälzenden Gases mindestens einen Schadstoff- und/oder Keimfilter auf, durch welchen das Gas während der Umwälzung strömt. Vorteilhaft ist hierbei, dass das Risiko einer Kontaminierung der Proben weiter verringert wird. Bevorzugt wird der mindestens eine Filter entlang der Gasführung angeordnet. Weiterhin ist es bevorzugt, den Filter von der Öffnung des Gehäuses aus zugänglich anzuordnen. Hierdurch wird die Wartung und der Austausch der Filter erleichtert und somit ganz allgemein die Handhabung der Erwärmungsvorrichtung verbessert.

In einer weiteren Ausführungsform weist der mindestens eine Filter verschiedene Dicken und/oder verschiedene Dichtigkeiten auf. Diese Eigenschaften haben Auswirkung auf die Geschwindigkeit, mit der das Gas den Filter durchströmt und bei entsprechender Anordnung der unterschiedlichen Filterstärken können somit unterschiedliche Strömungsgeschwindigkeiten des Gases ausgeglichen werden und die Gleichmäßigkeit des Gasstroms weiter verbessert werden.

In einer weiteren bevorzugten Ausführungsform weisen die Mittel zur Aufbereitung des umzuwälzenden Gases weiterhin eine Heizvorrichtung zur Erwärmung des Gases auf. Hierdurch ist es möglich, das umzuwälzende Gas auf eine bestimmte Temperatur zu erwärmen. Somit kann eine optimale Erwärmungstemperatur für die jeweiligen Proben erreicht werden. Bevorzugt wird die Heizvorrichtung entlang der Gasführung angeordnet. Hierbei kann die Heizvorrichtung beispielsweise als Heizschlange ausgebildet sein, an der das Gas innerhalb der Gasführung vorbeiströmt und dadurch erwärmt wird.

Zweckmäßigerweise weist die Erwärmungsvorrichtung weiterhin eine Kontrolleinrichtung auf, durch die die Temperatur im Lagerraum innerhalb einer bestimmten Temperaturspanne gehalten werden kann. Hierdurch wird sichergestellt, dass die Proben keinen zu hohen bzw. zu niedrigen Temperaturen ausgesetzt sind und die Probenqualität somit nicht beeinträchtigt wird.

Bevorzugterweise besteht das umzuwälzende Gas aus Luft. Dadurch wird der Aufbau und die Handhabung der Erwärmungsvorrichtung weiter vereinfacht. Zweckmäßigerweise wird bei dieser Ausführungsform als externes Gas Frischluft dem Umwälzkreislauf zugeführt. Hierbei ist es weiterhin besonders bevorzugt, dass der Anteil an Frischluft im Umwälzkreislauf ungefähr zehn Prozent entspricht.

In einer weiteren bevorzugten Ausführungsform ist die Erwärmungsvorrichtung integriert in einen Inkubator oder einen Klimaschrank ausgebildet. Hierdurch wird der Erwärmungsprozess der Proben weiter vereinfacht, da die Umlagerung der Proben in eine separate Erwärmungsvorrichtung nicht mehr durchgeführt werden muss ist. Zusätzlich wird der Platzbedarf innerhalb des Labors reduziert.

Weiterhin wird die der Erfindung zugrunde liegende Aufgabe durch ein Verfahren zur Erwärmung von Proben auf dem Gebiet der Life-Science, welche innerhalb eines Lagerraums angeordnet sind und bei welchen Gas zur Erwärmung der Proben auf diese geführt wird, dadurch gelöst, dass das Gas im Lagerraum umgewälzt wird, externes Gas zum Umwälzkreislauf zugeführt wird, das umzuwälzende Gas erwärmt wird, eine Filterung des umzuwälzenden Gases durch Keim- und Schadstofffilter durchgeführt wird und ein Teil des umzuwälzenden Gases aus dem Umwälzkreislauf wieder ausgeführt wird. Zur Erreichung einer kontinuierlichen Erwärmung werden diese Schritte wiederholt.

In einer bevorzugten Verfahrensvariante wird das externe Gas vor dem Zuführen zum Umwälzkreislauf mittels Keim- und Schadstofffiltern gefiltert. Hierdurch wird das Risiko einer Kontamination der Proben weiter verringert.

Weiterhin ist es bevorzugt, die Zuführung von externem Gas unter vorherbestimmten Mischverhältnissen durchzuführen. Somit kann ein optimales Gasgemisch zur Erwärmung der Proben erreicht werden.

In einer weiteren bevorzugten Verfahrensvariante besteht das Gas im Umwälzkreislauf aus Luft. Entsprechend ist es weiterhin bevorzugt, dass das externe Gas aus Frischluft besteht. Bei dieser Verfahrensvariante ist es weiterhin besonders bevorzugt, dass der Anteil an Frischluft im Umwälzkreislauf im Wesentlichen zehn Prozent beträgt.

Nachfolgend wird die Erfindung anhand in den Zeichnungen dargestellter Ausführungsbeispiele weiter beschrieben. Es zeigen schematisch:
- Fig. 1: eine Seitenansicht einer ersten Erwärmungsvorrichtung mit im Gehäuse integrierter Gasumwälzungseinrichtung;
- Fig. 2: die Ausführungsform aus Fig. 1 im Querschnitt;
- Fig. 3: eine Seitenansicht einer zweiten Erwärmungsvorrichtung mit auf das Gehäuse aufgesetzter Gasumwälzungseinrichtung; und
- Fig. 4: die Ausführungsform aus Fig. 3 im Querschnitt. ,

Bei den in den Figuren dargestellten, verschiedenen Ausführungsformen der Erfindung sind gleiche Teile mit gleichen Bezugszeichen versehen.

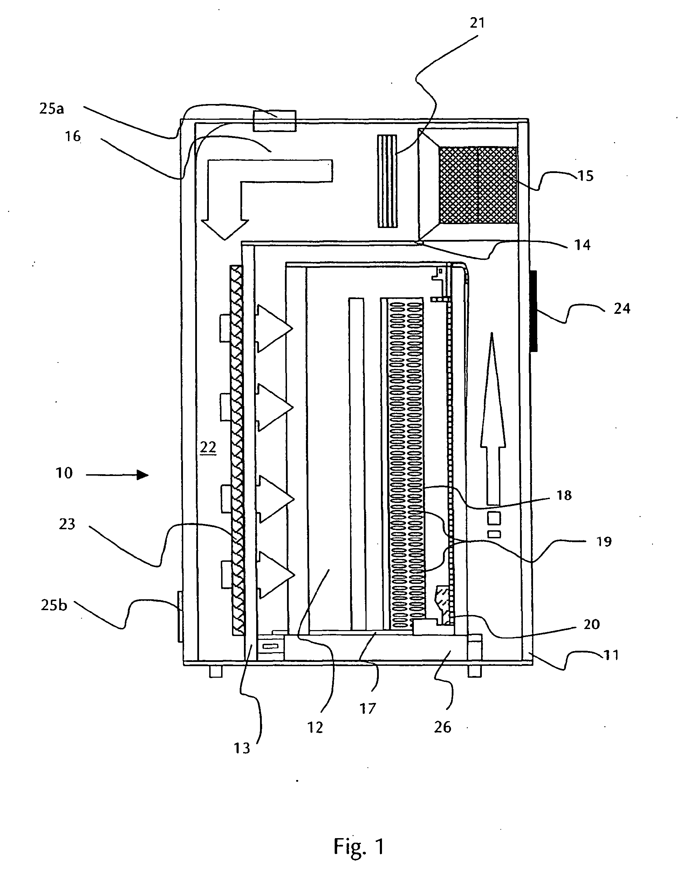

Fig. 1 zeigt eine Seitenansicht einer ersten Erwärmungsvorrichtung 10 mit einer im Gehäuse 11 integrierten Gasumwälzungsvorrichtung. Im Inneren des Gehäuses 11 ist ein Lagerraum 12 ausgebildet, dessen Boden und rechte Seite vom Gehäuse und dessen linke Seite von einer Innenwand 13 begrenzt wird. An seinem oberen Ende grenzt der Lagerraum 12 an eine Zwischendecke 14 und an ein Gebläse 15. Das Gebläse 15 saugt die im Lagerraum 12 befindliche Luft an und bläst sie in eine an die Decke des Gehäuses 11 angrenzende und als Schacht ausgebildete Gasleitung 16 ein. Die Gasleitung 16 verläuft in horizontaler Richtung. Die in Fig. 1 eingezeichneten Pfeile verdeutlichen die Strömungsrichtung der Luft und zeigen somit die Richtung des Umwälzkreislaufes der Erwärmungsvorrichtung 10 an.

Innerhalb des Lagerraums 12 und in dessen Bodenbereich ist eine als Karussell ausgebildete Transportplattform 17 vorhanden. Die kreisförmige Transportplattform 17 ist in ihrem Mittelpunkt drehbar gelagert. Auf der Transportplattform 17 ist eine Kassette 18 angeordnet, in deren Lagerfächern 19 sich Probenträger (hier nicht dargestellt) befinden. Die Kassette 18 kann durch Rotation der Transportplattform 17 innerhalb des Lagerraums 12 bewegt werden. Auf der rechten Seite an die Transportplattform 17 angrenzend ist eine aus der US 6,129,428 bekannte Bestückungsvorrichtung 20 angeordnet, durch die die Kassette 18 automatisch bestückbar ist.

Innerhalb der Gasleitung 16, direkt hinter dem Gebläse 15 in Strömungsrichtung ist eine Heizvorrichtung 21 angeordnet, durch die Luft, nachdem sie aus dem Gebläse 15 in die Gasleitung 16 eingeblasen wurde, erwärmt wird. Die Temperatur, auf die die Luft erwärmt wird, kann dabei durch Regulierung der Heizleistung der Heizvorrichtung 21 eingestellt werden. Nachdem die Luft die Heizvorrichtung 21 passiert, strömt sie weiter die Gasleitung 16 in horizontaler Richtung entlang, bis sie in eine vertikal ausgerichtete und als rechteckiger Schacht ausgebildete Gasführung 22 einströmt. Die Gasführung 22 wird auf der linken Seite von dem Gehäuse 11 und auf ihrer rechten Seite von der Innenwand 13 begrenzt. Die Gasführung 22 erstreckt sich über die gesamte Länge des Lagerraumes 12 und ist im Wesentlichen parallel zu den Kassetten 18 angeordnet. Durch dieses Anordnung wird sichergestellt, dass sich die in die Gasführung 22 einströmende Luft über die gesamte Länge der Kassetten 18 gleichmäßig verteilt. An der Innseite der Gasführung 22, die durch die Innenwand 13 begrenzt wird, ist ein Schadstoff- und/oder Keimfilter 23 angebracht. Zusätzlich sind in der Innenwand 13 Zuführungen (hier nicht dargestellt) angeordnet, durch die die Luft aus der Gasführung 22, nach Durchströmen des Filters 23 in den Lagerraum 12 einströmt. Wie in der Fig. 1 zu erkennen ist, strömt die Luft ungefähr in horizontaler Richtung in den Lagerraum 12 ein. Durch Drehen der Transportplattform 17 können die Kassetten 18 in Richtung der Gasführung 22 bewegt werden. Wenn die Kassetten 18 in der Nähe der Gasführung 22 sind, strömt die Luft aus der Gasführung 22 durch die Zuführung (hier nicht dargestellt) durch die Öffnungen der Lagerfächer 19 in die Kassette 18 ein und kann so die darin befindlichen Proben (hier nicht dargestellt) erwärmen. Die Kassetten 18 weisen üblicherweise mindestens eine Öffnung und einen an der gegenüberliegenden Seite angeordneten Ausgang auf, so dass die Luft die Kassetten 18 ungehindert durchströmen kann.

An der rechten Seitenwand des Gehäuses 11 ist weiterhin eine Gaseinlass 24 für die Zuführung von Frischluft in den Umwälzkreislauf angeordnet. Die Gaseinlass 24 ist mit einem Vorfilter (hier nicht dargestellt) versehen, durch den die Frischluft vor Eintritt in den Umwälzkreislauf gefiltert wird. Abgehend von der Gasleitung 16 ist in der Decke des Gehäuses 11 ein Gasauslass 25a angeordnet, durch den Luft aus dem Umwälzkreislauf abgelassen werden kann. Normalerweise wird der Auslass 25a so eingestellt, dass durch ihn in etwa die gleiche Menge Luft abgelassen wird, die durch den Einlass 24 in den Umwälzkreislauf eingelassen wird, so dass innerhalb der Erwärmungsvorrichtung 10 ein etwa konstanter Druck herrscht. Alternativ oder zusätzlich kann die Luft auch durch den Auslass 25b abgelassen werden, der in einer Außenwand des Gehäuses 11 im unteren Bereich der Gasführung 22 angeordnet ist. Der Boden des Lagerraumes 12 wird durch einen Schaltkasten 26 begrenzt, in dem elektrische Bauteile (hier nicht dargestellt) zur Steuerung und Regelung der Erwärmungsvorrichtung 10 angeordnet sind.

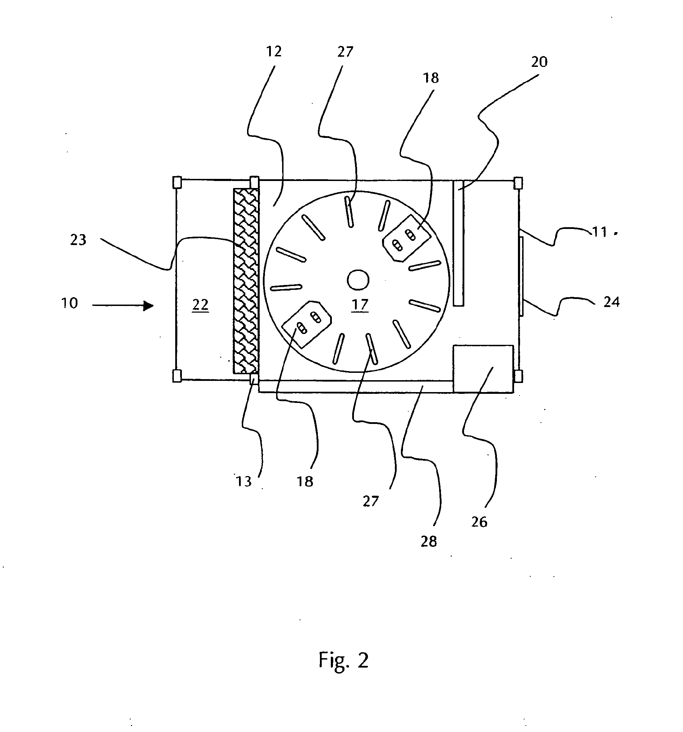

Fig. 2 zeigt die Ausführungsform aus Fig. 1 im Querschnitt. Es ist zu erkennen, dass die Transportplattform 17 Halterungen 27 aufweist, die zum Eingriff in die Kassetten 18 ausgebildet sind. Zwei Kassetten 18 sind auf der Transportplattform 17 gegenüberliegend angeordnet und greifen jeweils in eine Halterung 27 ein. Der mittlere Bereich der unteren Seitenwand des Gehäuses 11 ist als Tür 28 ausgebildet. Die Tür 28 kann aufgeschwenkt werden und gewährt somit Zugriff auf den Lagerraum 12.

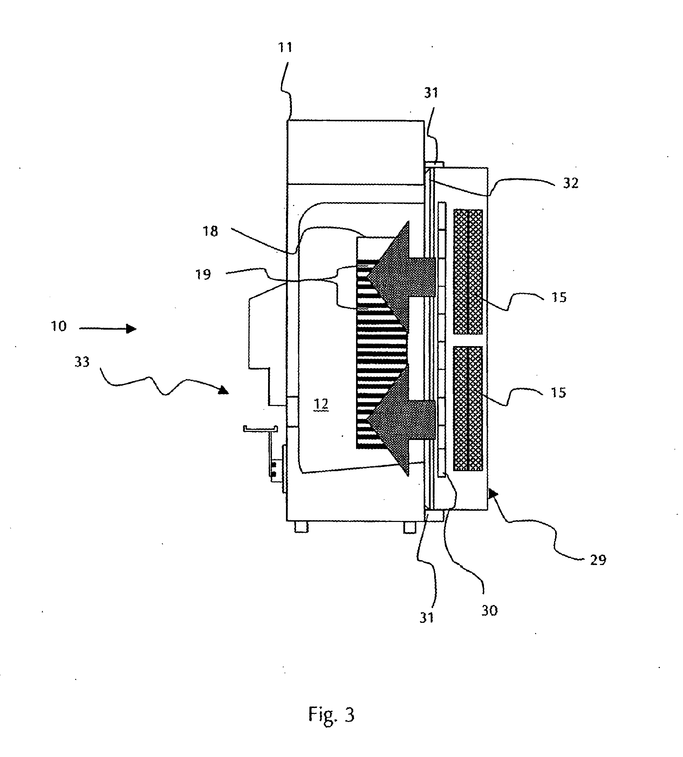

Fig. 3 zeigt eine zweite Ausführungsform einer Erwärmungsvorrichtung 10 in der Seitenansicht. Im Lagerraum 12 sind Kassetten 18 mit Lagerfächern 19 angeordnet. In den Lagerfächern 19 befinden sich Proben in Mikrotiterplatten (hier nicht dargestellt). Die rechte Seite des Gehäuses 11 weist eine Öffnung auf, die durch die auf das Gehäuse 11 aufgesetzte Gasumwälzungsvorrichtung 29 verschließbar ist. Im Inneren der Gasumwälzungsvorrichtung 29 sind Gebläse 15 und integrierte Heiz- und Filtervorrichtungen 30 angeordnet. Die Gasumwälzungsvorrichtung 29 ist durch Scharniere 31 am Gehäuse 11 angebracht und schwenkbar gelagert. Die Gasumwälzungsvorrichtung 29 ist dabei so angeordnet, dass sie die Öffnung des Gehäuses 11 im geschlossenen Zustand abdeckt. Zwischen der Gasumwälzung 29 und dem Gehäuse 11 ist zusätzlich eine Dichtungslage 32 angeordnet. An der linken Seitenwand des Gehäuses 11 ist weiterhin eine automatische Bestückungseinrichtung 33 angebracht, durch die die Kassetten 18 automatisch mit Probenträgern bestückt werden können. Weiterhin ist zu erkennen, dass die umgewälzte Luft aus der Gasumwälzungseinrichtung 29 ungefähr horizontal in den Lagerraum 12 einströmt und auch die Lagerfächer 19 der Kassetten 18 in horizontaler Richtung durchströmt.

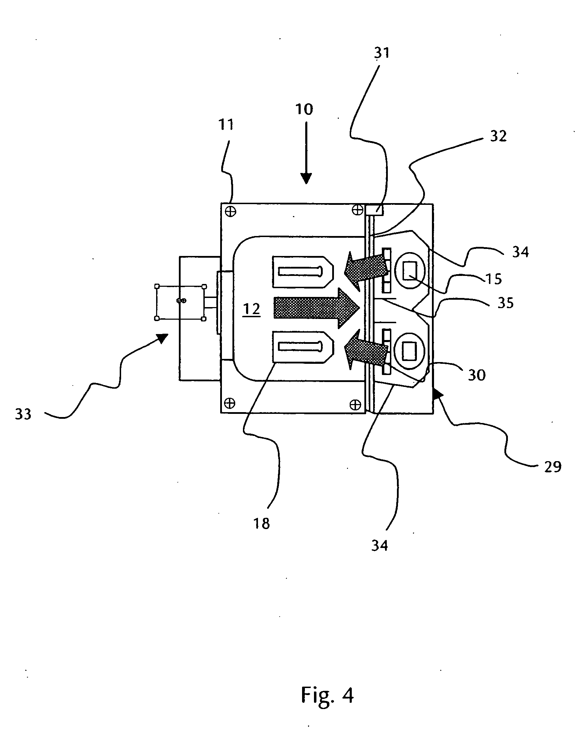

Fig. 4 zeigt die Ausführungsform aus Fig. 3 im Querschnitt. Es ist zu erkennen, dass im Inneren der Gasumwälzungseinrichtung 29 ein durch Innenwände 34 abgetrennter Bereich vorhanden ist, in dem die Gebläse 15 und die integrierten Heiz-/Filtervorrichtungen 30 angeordnet sind. Dieser durch die Innenwände 34 abgetrennte Bereich schließt an seinen Seiten bündig mit dem Lagerraum 12 ab. Im Inneren dieses Bereiches sind zwei weitere Zwischenwände 35 angeordnet. Die Innenwände 34 und die Zwischenwände 35 sind durchgehend vom Boden bis zur Decke ausgebildet. Die Zwischenwände 35 sind parallel zu den Seitenwänden des Gehäuses 11 ausgerichtet und beginnen am innenseitigen Ende der Gehäuseeinrichtung 29 und enden im freien Raum. Somit ist der Innenbereich der Gehäuseeinrichtung 29 in drei Unterbereiche unterteilt, die allerdings alle miteinander in Verbindung stehen und untereinander Luft austauschen können. Die Pfeile deuten die Strömungsrichtung der Luft an. Luft wird von den Gebläsen 15 angesaugt und danach wieder in Richtung des Lagerraumes 12 ausgeblasen. Dabei durchströmt es erst die integrierte Heiz-/Filtervorrichtung 30 und strömt dann weiter durch Zuführungen (hier nicht dargestellt) in der Dichtungslage 32 in den Lagerraum 12. Die beiden Kassetten 18 sind jeweils in der Nähe einer Zuführung angeordnet und werden von der Luft in horizontaler Richtung durchströmt. Die Luft strömt durch die Kassetten 18 hindurch, sammelt sich im hinteren Bereich des Lagerraums 12 und strömt dann wieder in etwa mittig durch eine Lücke zwischen den beiden Kassetten 18 in die Gasumwälzungseinrichtung 29 zurück.

## Patentansprüche

1. Erwärmungsvorrichtung (10) für Proben auf dem Gebiet der Life-Science mit Kassetten (18) mit horizontal ausgerichteten Lagerfächern(19) zur Lagerung von Probenträgern, bei welcher Gas zur Erwärmung der Proben auf die Kassetten (18) geführt ist, mit einem Gehäuse (11) mit einem darin angeordneten Lagerraum (12), der zur Zu- und Abfuhr von Gas und zur Lagerung der Kassetten (18) ausgebildet ist, und mit einer Gasumwälzungseinrichtung (29) zur Umwälzung des Gases im Lagerraum (12),
**dadurch gekennzeichnet,**
**dass** die Kassetten (18) mittels Bewegungsmitteln ortsverschiebbar und durch eine Steuereinrichtung in Richtung der Gasführung bewegbar sind, wobei die Bewegungsmittel mindestens eine horizontal verschwenkbar oder rotierbare Kassettenlagerplattform umfassen, und dass die Bewegungsmittel eine Transporteinrichtung (33) zum automatischen Einbringen und Entfernen der Probenträger in den Lagerraum (12) umfassen, wobei die Transporteinrichtung (33) zur Verschiebung der Probenträger senkrecht und parallel zur Rotationsachse der Kassettenlagerplattform ausgebildet ist.

2. Erwärmungsvorrichtung (10) gemäß Anspruch 1,
**dadurch gekennzeichnet,**
**dass** die Gasumwälzungseinrichtung (29) eine Zuführung zur Beimischung von externem Gas zum Umwälzkreislauf sowie eine Abführung zur Abfuhr eines Teils des umzuwälzenden Gases aus dem Umwälzkreislauf aufweist.

3. Erwärmungsvorrichtung (10) gemäß einem der vorhergehenden Ansprüche,
**dadurch gekennzeichnet,**
**dass** die Gasumwälzungseinrichtung (29) Mittel zur Aufbereitung des umzuwälzenden Gases aufweist.

4. Erwärmungsvorrichtung (10) gemäß einem der vorhergehenden Ansprüche,
**dadurch gekennzeichnet,**
**dass** die Gasumwälzungsvorrichtung (29) eine Gasführung (22) aufweist, die das Gas entlang der gesamten Länge der mindestens einen Kassette führt und in im Wesentlichen horizontaler Ausrichtung in die Kassette einleitet.

5. Erwärmungsvorrichtung (10) gemäß Anspruch 4,
**dadurch gekennzeichnet,**
**dass** die Gasführung (22) als Schacht ausgebildet ist, der an den Lagerraum (12) angrenzt und sich über die Länge des Lagerraums (12) erstreckt.

6. Erwärmungsvorrichtung (10) gemäß einem der vorhergehenden Ansprüche,
**dadurch gekennzeichnet,**
**dass** die Gasumwälzungseinrichtung (29) in das Gehäuse (11) integriert ausgebildet ist.

7. Erwärmungsvorrichtung (10) gemäß einem der Ansprüche 1 bis 5,
**dadurch gekennzeichnet,**
**dass** die Gasumwälzungseinrichtung (29) auf das Gehäuse (11) aufsetzbar ausgebildet und mittels Befestigungsmitteln, insbesondere Scharniere (31), Schrauben, Bolzen oder Schweißen, an dem Gehäuse (11) befestigbar ist.

8. Erwärmungsvorrichtung (10) gemäß Anspruch 7,
**dadurch gekennzeichnet,**
**dass** die Gasumwälzungseinrichtung (29) mittels Scharnieren (31) im Bereich der Öffnung des Gehäuses an diesem befestigt ist und zur schwenkbaren Verschließung der Öffnung ausgebildet ist.

9. Erwärmungsvorrichtung (10) gemäß einem der Ansprüche 4 bis 7,
**dadurch gekennzeichnet,**
**dass** die Gasführung versetzt von der Öffnung des Gehäuses (11) angeordnet ist.

10. Erwärmungsvorrichtung (10) gemäß einem der vorhergehenden Ansprüche,
**dadurch gekennzeichnet,**
**dass** die Gasumwälzeinrichtung (29) einen Antrieb zur Umwälzung des Gases des Lagerraums (12), insbesondere ein Gebläse (15), aufweist.

11. Erwärmungsvorrichtung (10) gemäß einem der vorhergehenden Ansprüche,
**dadurch gekennzeichnet,**
**dass** die Steuereinrichtung die Geschwindigkeit und/oder die Frequenz der Verschiebung der Kassetten (18) in Abhängigkeit der Strömungsgeschwindigkeit des auf die Kassetten (18) geführten Gases steuert.

12. Erwärmungsvorrichtung(10) gemäß einem der vorhergehenden Ansprüche,
**dadurch gekennzeichnet,**
**dass** die Kassettenlagerplattform als Drehscheibe ausgebildet ist.

13. Erwärmungsvorrichtung (10) gemäß einem der vorhergehenden Ansprüche,
**dadurch gekennzeichnet,**
**dass** die Kassetten (18) auf der Kassettenlagerplattform im Wesentlichen gleichmäßig verteilt angeordnet sind.

14. Erwärmungsvorrichtung (10) gemäß einem der Ansprüche 2 bis 13,
**dadurch gekennzeichnet,**
**dass** das externe Gas dem Umgebungsgas des Lagerraumes (12) unter einem vorherbestimmten Mischungsverhältnis beigemischt ist.

15. Erwärmungsvorrichtung (10) gemäß einem der Ansprüche 2 bis 14,
**dadurch gekennzeichnet,**
**dass** die Zuführung für das externe Gas einen Schadstoff- und/oder Keimfilter zur Filterung des externen Gases aufweist.

16. Erwärmungsvorrichtung (10) gemäß einem der Ansprüche 3 bis 15,
**dadurch gekennzeichnet,**
**dass** die Mittel zur Aufbereitung des umzuwälzenden Gases mindestens einen Schadstoff- und/oder Keimfilter, welchen das Gas während der Umwälzung durchströmt, aufweist.

17. Erwärmungsvorrichtung (10) gemäß Anspruch 16,
**dadurch gekennzeichnet,**
**dass** der mindestens eine Filter entlang der Gasführung angeordnet ist.

18. Erwärmungsvorrichtung (10) gemäß Anspruch 16 oder 17,
**dadurch gekennzeichnet,**
**dass** der mindestens eine Filter mit verschiedenen Dicken und/oder verschiedenen Dichtigkeiten ausgebildet ist.

19. Erwärmungsvorrichtung (10) gemäß einem der Ansprüche 16 bis 18,
**dadurch gekennzeichnet,**
**dass** der mindestens eine Filter von der Öffnung des Gehäuses (11) aus zugänglich angeordnet ist.

20. Erwärmungsvorrichtung (10) gemäß einem der Ansprüche 3 bis 19,
**dadurch gekennzeichnet,**
**dass** die Mittel zur Aufbereitung des umzuwälzenden Gases eine Heizvorrichtung zur Erwärmung des Gases während der Umwälzung umfassen.

21. Erwärmungsvorrichtung (10) gemäß Anspruch 20,
**dadurch gekennzeichnet,**
**dass** die Heizvorrichtung entlang der Gasführung angeordnet ist.

22. Erwärmungsvorrichtung (10) gemäß einem der vorhergehenden Ansprüche,
**dadurch gekennzeichnet,**
**dass** eine Kontrolleinrichtung vorhanden ist, durch die die Temperatur innerhalb des Lagerraums unterhalb und/oder oberhalb einer vorherbestimmten Temperatur haltbar ist.

23. Erwärmungsvorrichtung (10) gemäß einem der vorhergehenden Ansprüche,
**dadurch gekennzeichnet,**
**dass** das umzuwälzende Gas aus Luft besteht.

24. Erwärmungsvorrichtung (10) gemäß einem der vorhergehenden Ansprüche,
**dadurch gekennzeichnet,**
**dass** die Erwärmungsvorrichtung (10) integriert in einen Inkubator oder einen Klimaschrank ausgebildet ist.

25. Verfahren zur Erwärmung von Proben auf dem Gebiet der Life-Science, welche innerhalb eines Lagerraumes (12) angeordnet sind, bei welchem Gas zur Erwärmung der Proben auf diese in einer Erwärmungsvorrichtung (10) gemäß einem der Ansprüche 1 bis 24 geführt wird, und bei dem die Kassetten zur gleichmäßigen Durchströmung in Richtung der Gasführung bewegt werden, wobei folgende Schritte durchgeführt werden:
a) Umwälzen des Gases im Lagerraum (12);
b) Zuführen von externem Gas zum Umwälzkreislauf;
c) Erwärmung des umzuwälzenden Gases;
d) Filterung des umzuwälzenden Gases mit Keim- und/oder Schadstofffiltern;
e) Abführen eines Teils des umzuwälzenden Gases aus dem Umwälzkreislauf; und
f) Wiederholung der Schritte a) bis e).

26. Verfahren gemäß Anspruch 25,
**dadurch gekennzeichnet,**
**dass** das externe Gas vor dem Zuführen zum Umwälzkreislauf analog zu Schritt d) gefiltert wird.

27. Verfahren gemäß Anspruch 25 oder 26,
**dadurch gekennzeichnet,**
**dass** das die Zuführung von externem Gas unter vorherbestimmten Mischverhältnissen durchgeführt wird.

28. Verfahren gemäß einem der Ansprüche 25 bis 27,
**dadurch gekennzeichnet,**
**dass** das Umgebungsgas des Lagerraums aus Luft besteht.

29. Verfahren gemäß Anspruch 28,
**dadurch gekennzeichnet,**
**dass** das externe Gas aus Frischluft besteht.

30. Verfahren gemäß Anspruch 29,
**dadurch gekennzeichnet,**
**dass** der Anteil an Frischluft im Umwälzkreislauf im Wesentlichen 10% beträgt.

## Claims

1. A heating apparatus (10) for samples in the field of life science, comprising cassettes (18) with horizontally aligned storage compartments (19) for storing sample holders in which gas for heating the samples is guided onto the cassettes (18), a housing (11) with a storage chamber (12) which is arranged therein and is formed for supplying and discharging gas and for storing the cassettes (18), and a gas circulation device (29) for circulating the gas in the storage chamber (12), **characterized in that** the cassettes (18) are locally displaceable by means of motion means and can be moved by a control device in the direction of the gas flow control, with the motion means comprising at least one cassette storage platform which is horizontally swivelable or rotatable, and that the motion means comprise a transport device (33) for the automatic introduction of the sample holders into the storage chamber (12) and the removal therefrom, with the transport device (33) being arranged for displacing the sample holder perpendicular and parallel to the rotational axis of the cassette storage platform.

2. A heating apparatus (10) according to claim 1, **characterized in that** the gas circulation device (29) comprises a feed for admixing external gas to the circulation and a discharge for removing a portion of the circulated gas from the circulation.

3. A heating apparatus (10) according to one of the preceding claims, **characterized in that** the gas circulation device (29) comprises means for the treatment of the gas to be circulated.

4. A heating apparatus (10) according to one of the preceding claims, **characterized in that** the gas circulation apparatus (29) comprises a gas flow control (22) which guides the gas along the entire length of the at least one cassette and introduces the gas in a substantially horizontal alignment into the cassette.

5. A heating apparatus (10) according to claim 4, **characterized in that** the gas flow control (22) is arranged as a chute which is adjacent to the storage chamber (12) and extends over the length of the storage chamber (12).

6. A heating apparatus (10) according to one of the preceding claims, **characterized in that** the gas circulation device (29) is arranged to be integrated in the housing (11).

7. A heating apparatus (10) according to one of the claims 1 to 5, **characterized in that** the gas circulation device (29) is arranged to be placeable on the housing (11) and can be fastened by means of fastening means, especially hinges (31), screws, bolts or welding.

8. A heating apparatus (10) according to claim 7, **characterized in that** the gas circulation device (29) is fastened to the area of the opening of the housing by means of hinges (31) and is arranged for swivelably sealing the opening.

9. A heating apparatus (10) according to one of the claims 4 to 7, **characterized in that** the gas flow control is arranged offset from the opening of the housing (11).

10. A heating apparatus (10) according to one of the preceding claims, **characterized in that** the gas circulation device (29) comprises a drive for circulating the gas of the storage chamber (12), especially a blower (15).

11. A heating apparatus (10) according to one of the preceding claims, **characterized in that** the control device controls the speed and/or the frequency of the displacement of the cassettes (18) depending on the flow speed of the gas guided to the cassettes (18).

12. A heating apparatus (10) according to one of the preceding claims, **characterized in that** the cassette storage platform is arranged as a rotating disk.

13. A heating apparatus (10) according to one of the preceding claims, **characterized in that** the cassettes (18) on the cassette storage platform are arranged distributed in a substantially even manner.

14. A heating apparatus (10) according to one of the claims 2 to 13, **characterized in that** the external gas is admixed to the ambient gas of the storage chamber (12) under a predetermined mixing ratio.

15. A heating apparatus (10) according to one of the claims 2 to 14, **characterized in that** the feed for the external gas comprises a pollutant and/or germ filter for filtering the external gas.

16. A heating apparatus (10) according to one of the claims 3 to 15, **characterized in that** the means for treating the gas to be circulated comprises at least one pollutant and/or germ filter, through which the gas flows during circulation.

17. A heating apparatus (10) according to claim 16, **characterized in that** the at least one filter is arranged along the gas flow control.

18. A heating apparatus (10) according to claim 16 or 17, **characterized in that** the at least one filter is arranged with different thicknesses and/or different tightness.

19. A heating apparatus (10) according to one of the claims 16 to 18, **characterized in that** the at least one filter is arranged so as to be accessible from the opening of the housing (11).

20. A heating apparatus (10) according to one of the claims 3 to 19, **characterized in that** the means for treating the gas to be circulated comprise a heating apparatus for heating the gas during circulation.

21. A heating apparatus (10) according to claim 20, **characterized in that** the heating apparatus is arranged along the gas flow control.

22. A heating apparatus (10) according to one of the preceding claims, **characterized in that** a checking device is present through which the temperature within the storage chamber can be kept beneath and/or above a predetermined temperature.

23. A heating apparatus (10) according to one of the preceding claims, **characterized in that** the gas to be circulated consists of air.

24. A heating apparatus (10) according to one of the preceding claims, **characterized in that** the heating apparatus (10) is integrated in an incubator or a conditioning cabinet.

25. A method for heating samples in the field of life science which are arranged within a storage chamber (12), in which gas is guided for heating the samples on the same in a heating apparatus (10) according to one of the claims 1 to 24, and in which the cassettes are moved for even flow through in the direction of gas flow control, with the following steps being performed:
a) circulation of the gas in the storage chamber (12);
b) supply of external gas to the circulation;
c) heating of the gas to be circulated;
d) filtering of the circulated gas with germ and/or pollutant filters;
e) removal of a portion of the circulated gas from the circulation, and
f) repetition of the steps a) through e).

26. A method according to claim 25, **characterized in that** the external gas is filtered prior to the supply to the circulation in analogy to step d).

27. A method according to claim 25 or 26, **characterized in that** the supply of external gas is performed under predetermined mixing ratios.

28. A method according to one of the claims 25 to 27, **characterized in that** the ambient gas of the storage chamber consists of air.

29. A method according to claim 28, **characterized in that** the external gas consists of fresh air.

30. A method according to claim 29, **characterized in that** the share of fresh air in the circulation is substantially 10%.

## Revendications

1. Dispositif de chauffage (10) pour des échantillons dans le domaine des sciences de la vie, avec des cassettes (18) comportant des compartiments de stockage (19) horizontaux pour déposer des porte-échantillons, dans lequel du gaz de chauffage est amené vers les cassettes (18), avec un boîtier (11) comportant à l'intérieur un espace de stockage (12) conçu en vue de l'arrivée et de l'extraction de gaz et pour le stockage des cassettes (18), et avec un dispositif de mise en circulation du gaz (29) pour faire circuler le gaz dans l'espace de stockage (12), **caractérisé en ce que** les cassettes (18) peuvent être déplacées au moyen de moyens de déplacement et amenées par un dispositif de commande dans la direction de l'arrivée de gaz, les moyens de déplacement comprenant au moins une plate-forme porte-cassettes qui peut pivoter horizontalement et tourner, et **en ce que** les moyens de déplacement comprennent un dispositif de transport (33) pour l'introduction et le retrait automatiques des porte-échantillons dans l'espace de stockage (12), lequel dispositif de transport (33) est conçu en vue d'une translation des porte-échantillons perpendiculairement et parallèlement à l'axe de rotation de la plate-forme porte-cassettes.

2. Dispositif de chauffage (10) selon la revendication 1, **caractérisé en ce que** le dispositif de mise en circulation du gaz (29) comprend une arrivée pour l'ajout de gaz externe au circuit de circulation ainsi qu'une extraction pour l'évacuation d'une partie du gaz à mettre en circulation hors du circuit de circulation.

3. Dispositif de chauffage (10) selon l'une des revendications précédentes, **caractérisé en ce que** le dispositif de mise en circulation du gaz (29) comprend des moyens pour la préparation du gaz à mettre en circulation.

4. Dispositif de chauffage (10) selon l'une des revendications précédentes, **caractérisé en ce que** le dispositif de mise en circulation du gaz (29) comprend une arrivée de gaz (22) qui guide le gaz sur toute la longueur de l'au moins une cassette et conduit dans la cassette selon une orientation sensiblement horizontale.

5. Dispositif de chauffage (10) selon la revendication 4, **caractérisé en ce que** l'arrivée de gaz (22) est conçue comme une gaine limitrophe de l'espace de stockage (12) et qui s'étend sur la longueur de l'espace de stockage (12).

6. Dispositif de chauffage (10) selon l'une des revendications précédentes, **caractérisé en ce que** le dispositif de mise en circulation du gaz (29) est intégré dans le boîtier (11).

7. Dispositif de chauffage (10) selon l'une des revendications 1 à 5, **caractérisé en ce que** le dispositif de mise en circulation du gaz (29) est conçu pour pouvoir être posé sur le boîtier (11) et fixé sur le boîtier (11) au moyen de moyens de fixation, en particulier de charnières (31), de vis, de boulons ou de soudures.

8. Dispositif de chauffage (10) selon la revendication 7, **caractérisé en ce que** le dispositif de mise en circulation du gaz (29) est fixé au boîtier au moyen de charnières (31) au niveau de l'ouverture du boîtier et est conçu en vue d'une fermeture pivotante de l'ouverture.

9. Dispositif de chauffage (10) selon l'une des revendications 4 à 7, **caractérisé en ce que** l'arrivée de gaz est décalée par rapport à l'ouverture du boîtier (11).

10. Dispositif de chauffage (10) selon l'une des revendications précédentes, **caractérisé en ce que** le dispositif de mise en circulation du gaz (29) possède un propulseur pour la mise en circulation du gaz de l'espace de stockage (12), en particulier une soufflerie (15).

11. Dispositif de chauffage (10) selon l'une des revendications précédentes, **caractérisé en ce que** le dispositif de commande régule la vitesse et/ou la fréquence de déplacement des cassettes (18) en fonction de la vitesse d'écoulement du gaz amené sur les cassettes (18).

12. Dispositif de chauffage (10) selon l'une des revendications précédentes, **caractérisé en ce que** la plate-forme porte-cassettes est conformée comme un disque tournant.

13. Dispositif de chauffage (10) selon l'une des revendications précédentes, **caractérisé en ce que** les cassettes (18) sont disposées sur la plate-forme porte-cassettes selon une répartition sensiblement régulière.

14. Dispositif de chauffage (10) selon l'une des revendications 2 à 13, **caractérisé en ce que** le gaz externe est ajouté au gaz ambiant de l'espace de stockage (12) selon une proportion de mélange définie.

15. Dispositif de chauffage (10) selon l'une des revendications 2 à 14, **caractérisé en ce que** l'arrivée du gaz externe contient un filtre à polluants et/ou à micro-organismes pour la filtration du gaz externe.

16. Dispositif de chauffage (10) selon l'une des revendications 3 à 15, **caractérisé en ce que** les moyens pour la préparation du gaz à mettre en circulation comprennent au moins un filtre à polluants et/ou à micro-organismes qui est parcouru par le gaz pendant la mise en circulation.

17. Dispositif de chauffage (10) selon la revendication 16, **caractérisé en ce que** l'au moins un filtre est disposé le long de l'arrivée de gaz.

18. Dispositif de chauffage (10) selon la revendication 16 ou 17, **caractérisé en ce que** l'au moins un filtre est conçu avec différentes épaisseurs et/ou différentes étanchéités.

19. Dispositif de chauffage (10) selon l'une des revendications 16 à 18, **caractérisé en ce que** l'au moins un filtre est disposé de façon à être accessible par l'ouverture du boîtier (11).

20. Dispositif de chauffage (10) selon l'une des revendications 3 à 19, **caractérisé en ce que** les moyens pour préparer le gaz à mettre en circulation comprennent un dispositif chauffant destiné à chauffer le gaz pendant sa mise en circulation.

21. Dispositif de chauffage (10) selon la revendication 20, **caractérisé en ce que** le dispositif chauffant est disposé le long de l'arrivée de gaz.

22. Dispositif de chauffage (10) selon l'une des revendications précédentes, **caractérisé en ce qu'**il est prévu un dispositif de contrôle qui peut maintenir la température à l'intérieur de l'espace de stockage en dessous et/ou au-dessus d'une température prédéterminée.

23. Dispositif de chauffage (10) selon l'une des revendications précédentes, **caractérisé en ce que** le gaz à mettre en circulation se compose d'air.

24. Dispositif de chauffage (10) selon l'une des revendications précédentes, **caractérisé en ce que** le dispositif de chauffage (10) est intégré dans un incubateur ou une armoire climatique.

25. Procédé pour le chauffage d'échantillons dans le domaine des sciences de la vie qui sont disposés à l'intérieur d'un espace de stockage (12), dans lequel du gaz est amené aux éprouvettes afin de les chauffer dans un dispositif de chauffage (10) selon l'une des revendications 1 à 24, et dans lequel les cassettes sont déplacées vers l'arrivée de gaz en vue d'une circulation uniforme, dans lequel les étapes suivantes sont exécutées :
a) mise en circulation du gaz dans l'espace de stockage (12) ;
b) arrivée de gaz externe vers le circuit de circulation ;
c) chauffage du gaz à mettre en circulation ;
d) filtration du gaz à mettre en circulation au moyen de filtres à micro-organismes et/ou à polluants ;
e) extraction d'une partie du gaz à mettre en circulation hors du circuit de circulation ; et
f) répétition des étapes a) à e).

26. Procédé selon la revendication 25, **caractérisé en ce que** le gaz externe est filtré d'une manière analogue à l'étape d) avant d'être amené au circuit de circulation.

27. Procédé selon la revendication 25 ou 26, **caractérisé en ce que** l'arrivée de gaz externe est effectuée selon des rapports de mélange déterminés.

28. Procédé selon l'une des revendications 25 à 27, **caractérisé en ce que** le gaz ambiant de l'espace de stockage se compose d'air.

29. Procédé selon la revendication 28, **caractérisé en ce que** le gaz externe se compose d'air frais.

30. Procédé selon la revendication 29, **caractérisé en ce que** la proportion d'air frais dans le circuit de circulation est sensiblement de 10 %.
